# EUROPEAN PATENT SPECIFICATION

(11) **EP 0 863 778 B1**
(45) Date of publication and mention of the grant of the patent: **30.08.2000**
(21) Application number: 96938343.9
(22) Date of filing: 12.11.1996
(51) Int. Cl.: A61M 16/04, A61L 27/00

(54) **ENDOTRACHEAL TUBES WITH PULMONARY MEDICAMENTS**
ENDOTRACHEALTUBUS MIT LUNGENMEDIKAMENT
TUBAGES ENDOTRACHEAUX PORTEURS DE MEDICAMENTS DESTINES AUX AFFECTIONS PULMONAIRES

(30) Priority: 23.11.1995 GB 9524007
(43) Date of publication of application: 16.09.1998
(73) Proprietor: BRITANNIA PHARMACEUTICALS LIMITED, Redhill, Surrey RH1 6YS (GB)
(72) Inventor: Bangham, Alec Douglas, Great Shelford, Cambridge CB2 5EH (GB); Morley, Colin John, Great Shelford, Cambridge CB2 5EH (GB)
(74) Representative: Woodcraft, David Charles
(86) International application number: GB9602787
(87) International publication number: WO9718849

(56) References cited:
- EP-A- 0 348 967
- WO-A-91/12779
- WO-A-93/17744
- US-A- 3 610 247
- US-A- 4 678 466

## Description

### Background of the Invention

This invention relates to the administration of medicaments to the lungs and, in particular, provides a device for administration of a pulmonary medicament, such as a lung surfactant.

Various systems exist for administration of medicaments to the lungs, for example, in the case of asthma conditions, it is conventional practice to administer the medicament in a nebuliser. However, in the case of patients who are unable to participate actively in the treatment, such as new born babies, the medicament may be given by means of a syringe through a catheter, directly into the respiratory tract. One difficulty with patients who are being ventilated is that in order to administer the medicament, the patient has to be disconnected from the ventilator while the medicament is administered, and this is a potentially dangerous step in the case of critically ill patients.

It has become the practice in the case of babies who experience breathing difficulties, to administer medicaments, commonly known as lung surfactants, to alleviate respiratory distress syndrome. One artificial surfactant which has been successfully used is sold under the trade name "ALEC" and is a dry protein-free powder which is a mixture of dipalmitoylphosphatidylcholine (DPPC) and phosphatidylglycerol (PG) in the molar ratio of 7:3 of DPPC to PG. This phospholipid mixture has a melting point in water in the region of body temperature. It acts by forming a mono layer on the surface of the alveolae and reducing the surface tension of the air/water interface by a factor of about two-thirds. This facilitates the extension of the baby's air-water interface, by reducing the work required to extend the effective area of the lungs. At blood temperature and as a consequence of repeated compression and relaxation, the DPPC component in the mixture becomes progressively enriched to the point that it condenses out of the mixture as a solid phase which is rigid enough to help prevent the lung alveoli from collapsing (anti-oedema effect).

Although the above medicaments have proved to be exceptionally effective when properly placed, one difficulty has been in applying the medicament quickly enough and in minimising the time when the subject is not connected to the ventilator.

An object of the present invention is to overcome these difficulties and to provide more effective methods and apparatus for administering lung surfactants and other pulmonary medicaments.

US-A-3610247 describes an endotracheal tube having an inflatable cuff which is coated with a local anaesthetic to facilitate introduction of the tube into a patient.

EP-A-0348967 is concerned with a synthetic lung preparation. It mentions that a nebulized suspension or drug mixture may be introduced into the inspired gas in an endotracheal tube.

According to one aspect of the present invention, there is provided a device for administering a lung surfactant directly to the lungs of a subject which comprises an endotracheal tube adapted for introduction into the trachea, said tube supporting distally a lung surfactant, thus permitting intubation of the subject while simultaneously administering lung surfactant directly to the subject's lungs.

The pulmonary medicament is generally a solid material, preferably one which has been deposited from suspension or solution. For example, the endotracheal tube may be dipped into a suspension or concentrated solution of the medicament, removed from the solution or suspension and then dried to deposit the medicament on the distal end surface or surfaces of the tubes.

The medicament is deposited on surfaces of an endotracheal tube which is connectable to a ventilator. Thus, in the case of a breathless infant, the attending physician can immediately intubate the patient with an endotracheal tube carrying lung surfactant. In this way, the lung surfactant is administered directly into the respiratory tract of the infant. The endotracheal tube is used for ventilating the infant.

The method and apparatus of the present invention is particularly suitable for administration of a lung surfactant to both neonatal infants, children and adults experiencing respiratory distress. The preferred lung surfactant is available under the trade mark "ALEC" and its composition and use is described in the paper by Bangham et al in Colloids & Surfaces 10, (1984), pages 337 to 341, and in the paper entitled "Ten Centre Trial of Artificial Surfactant in Very Premature Babies", British Medical Journal, 18th April 1987, vol. 294, pages 991 to 996. 'ALEC' lung surfactant is marketed by Britannia Pharmaceuticals Limited, Forum House, Brighton Road, Redhill, Surrey, England. Other commercially available lung surfactants may be administered in accordance with this invention. Examples are available under the trade names "Exosurf", "Curosurf", "Survanta", "Infasurf", "Bles", KL4", "Alveofact" and "Surfactant TA".

In the case of the lung surfactant described above and sold under the trade mark 'ALEC', an amount of approximately 2 mg is theoretically sufficient to facilitate the rapid extension of the child's lungs. However, there is no real disadvantage in administering a large excess of the lung surfactant since it is an essentially natural product. In practice, therefore, a sufficient amount or excess may be applied to the surfaces of the endotracheal tube, depending upon whether infants, children or adults are to be treated. Typically, the amount may be from about 2 to 20 mg

### Brief Description of the Drawings

Figure 1 is a side elevation of an endotracheal tube in accordance with the invention; and
Figure 2 is a diagrammatic view illustrating a method of employing the endotracheal tube.

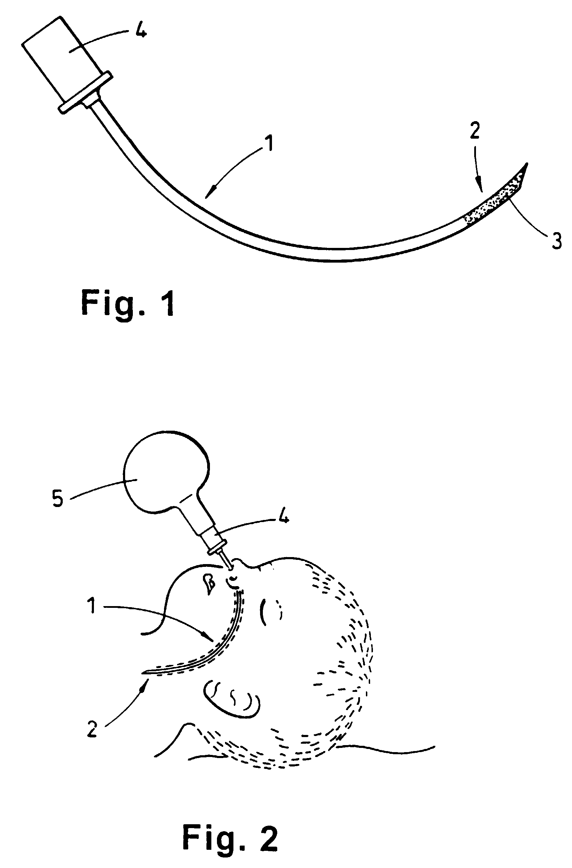

Referring to the drawings, Figure 1 shows an endotracheal tube comprising a plastics tube (1), e.g. about 3.0∼4.0 mms diameter, for introducing into the infants' nasal or oral passage. One end (2) of the plastic tube (the distal end) carries an external and/or internal coating (3) of a lung surfactant which has been deposited onto the surface(s) of the tube. The lung surfactant coating may conveniently extend over the distal 5 to 15 mms, e.g. 10 mms, of the tube. At the opposite end of the tube (1), a connector (4) is attached to the tube, e.g. by a frictional fit or by heat or solvent welding.

Figure 2 shows diagrammatically, the method of using the endotracheal tube. The distal end (2) of the tube is introduced through the nose or mouth and the connector (4) is attached to a resuscitation bag or ventilator (5). With the tube in place, the infant is intubated until he is able to breathe unaided. It will be appreciated that the endotracheal tube of the invention enables the patient, e.g. a premature neonate, to be intubated while simultaneously administering a pulmonary surfactant.

The surfactant is conveniently deposited on the tube from a solvent solution and any medically acceptable solvent which can be easily volatilised may be used. In the case of the lung surfactant referred to above, chloroform or ethanol/ethyl ether mixtures (e.g. ethanol/ether in a 1:1 mixture) are examples of suitable solvents. The resulting concentrated solution may be filtered through a bacterial filter prior to application to the tube, e.g. by dipping. After dipping the tube in the concentrated solution of the medicament, the tube may be dried, e.g. in a stream of hot sterile air or vacuum dried, to remove the solvent at a temperature which does not harm the active material. In the case of lung surfactant containing protein, this should be at a temperature below about 45°C since protein tends to be degraded at higher temperatures.

In the case of lung surfactants of the kind referred to above, the material dries to form a smear having a consistency similar to that of dry, hard soap (a Coagel). This smear has the additional advantage of acting as a lubricant for the introduction of the tube into the respiratory passage of the patient.

Although not so convenient, it is possible to apply the pulmonary medicament to an impregnated mop, wick or rod which can then be passed through an endotracheal tube, thereby replenishing the inner surface of the endotracheal tube. Such a technique may require only a short period of interruption of the ventilation of the patient, particularly if the endotracheal tube is suitably adapted to receive a catheter, mop or other elongate member without disconnection of the ventilator.

While the invention has been described with particular reference to its use for administration of lung surfactants to infants, it would also be applicable to cases of acute drowning, pneumonia, pulmonary oedema, and adult respiratory distress (ARDS).

It is possible to use the apparatus of this invention for veterinary purposes, for example, neonatal horses and pigs sometimes suffer from breathing difficulties due to deficiency of lung surfactant. The endotracheal tube or other elongate flexible applicator would, of course, need to be considerably longer and, in the case of an endotracheal tube of larger bore, but the technique is essentially similar.

## Claims

1. A device for administering a lung surfactant directly to the lungs of a subject which comprises an endotracheal tube (1) adapted for introduction into the trachea, characterised in that said tube supports distally (2) a lung surfactant (3), thus permitting intubation of the subject while simultaneously administering lung surfactant directly to the subjects lungs.

2. A device according to claim 1 wherein the surfactant is a powdery or particulate deposit on the distal inner and/or outer surface of the tube.

3. A device according to claim 1 or 2 wherein the distal portion carries a layer of a natural or synthetic solid lung surfactant in an amount of from 2 to 20 mg.

4. A tube according to any one of the preceding claims wherein the lung surfactant is a mixture of dipalmitoylphosphatidylcholine (DPPC) and phosphatidylglycerol (PG).

5. A method of producing a device as claimed in any one of the preceding claims comprising:
providing an endotracheal tube,
applying a concentrated solution or dispersion of lung surfactant to the tube, and
causing the continuous phase to deposit a layer of solid surfactant on or in the tube.

## Patentansprüche

1. Vorrichtung zur direkten Verabreichung eines Lungen-Surfactants an die Lungen einer Person, welche einen Endotrachealtubus (1) umfaßt, der für die Einführung in die Trachea vorgesehen ist, dadurch gekennzeichnet, daß dieser Tubus distal (2) ein Lungen-Surfactant (3) trägt und somit die Intubation der Person bei gleichzeitiger direkter Verabreichung von Lungen-Surfactant an die Lungen der Person ermöglicht.

2. Vorrichtung nach Anspruch 1, bei der das Surfactant eine pulverige oder partikuläre Abscheidung auf der distalen inneren und/oder äußeren Oberfläche des Tubus ist.

3. Vorrichtung nach Anspruch 1 oder 2, bei der der distale Teil eine Schicht aus einem natürlichen oder synthetischen festen Lungen-Surfactant in einer Menge von 2 bis 20 mg trägt.

4. Tubus nach einem der vorhergehenden Ansprüche, bei dem das Lungen-Surfactant ein Gemisch aus Dipalmitoylphosphatidylcholin (DPPC) und Phosphatidylglycerol (PG) ist.

5. Verfahren zur Herstellung einer Vorrichtung, wie sie in einem der vorhergehenden Ansprüche beansprucht ist, umfassend:
die Bereitstellung eines Endotrachealtubus,
das Aufbringen einer konzentrierten Lösung oder Dispersion eines Lungen-Surfactants auf den Tubus und
das Bewirken, daß die kontinuierliche Phase eine Schicht aus festem Surfactant auf oder in dem Tubus abscheidet.

## Revendications

1. Un dispositif pour administrer un agent tensioactif pulmonaire directement dans les poumons d'un sujet, dispositif qui comprend un tube endotrachéal (1) adapté pour l'introduction dans la trachée, caractérisé en ce que ledit tube supporte à sa partie terminale (2) un agent tensioactif pulmonaire (3) permettant ainsi l'intubation du sujet tout en administrant simultanément l'agent tensioactif pulmonaire directement dans les poumons du sujet.

2. Un dispositif selon la revendication 1, dans lequel l'agent tensioactif se présente sous la forme d'un dépôt poudreux ou de particules, déposé sur la surface intérieure et/ou extérieure de la partie terminale du tube.

3. Un dispositif selon la revendication 1 ou 2, dans lequel la partie terminale porte une couche d'agent tensioactif pulmonaire solide, naturel ou synthétique, en une quantité de 2 à 20 mg.

4. Un tube selon l'une quelconque des revendications précédentes, dans lequel l'agent tensioactif pulmonaire est un mélange de dipalmitoyl de glycérophospholipide à choline (CPPD) et d'ester phosphatidique du glycérol (GP).

5. Un procédé destiné à la production du dispositif revendiqué dans l'une quelconque des revendications précédentes comprenant :
- la fourniture d'un tube endotrachéal,
- l'application d'une solution concentrée ou d'une dispersion concentrée d'agent tensioactif pulmonaire dans le tube, et
- le fait de déposer, en phase continue, une couche d'agent tensioactif solide sur ou dans le tube.
